Europäisches Patentamt

European Patent Office

Office européen des brevets

(11) Veröffentlichungsnummer : **0 120 445**
**B1**

(12) **EUROPÄISCHE PATENTSCHRIFT**

(45) Veröffentlichungstag der Patentschrift :
23.11.89

(51) Int. Cl.⁴ : **A 61 M 1/34**

(21) Anmeldenummer : 84103072.9

(22) Anmeldetag : 21.03.84

(54) Verfahren und Vorrichtung zur selektiven extrakorporalen Abtrennung pathologischer und/oder toxischer Blutbestandteile.

(30) Priorität : 24.03.83 DE 3310727

(43) Veröffentlichungstag der Anmeldung :
03.10.84 Patentblatt 84/40

(45) Bekanntmachung des Hinweises auf die Patenterteilung : 23.11.89 Patentblatt 89/47

(84) Benannte Vertragsstaaten :
AT BE CH DE FR GB IT LI LU NL SE

(56) Entgegenhaltungen :
EP--A-- 0 041 350
EP--A-- 0 044 694
EP--A-- 0 074 610
GB--A-- 2 020 570
US--A-- 2 598 210
US--A-- 3 598 242
US--A-- 4 154 688
ARTIFICIAL ORGANS, Band 4, Nr. 3, August 1980, Seiten 205-207, International Society of Artificial Organs, Cleveland, Ohio, US; P.S. MALCHESKY et al.: "On-line separation of macromolecules by membrane filtration with cryogelation"

(73) Patentinhaber : B. Braun-SSC AG
Gerliswilstrasse 74
CH-6020 Emmenbrücke (CH)

(72) Erfinder : Rosskopf, Gerhard, Dr.
Heiligenbergstrasse 29
D-3501 Fuldabrück-Dörnhagen (DE)
Erfinder : Seidel, Dietrich, Dr.
Dahlmannstrasse 23
D-3400 Göttingen (DE)
Erfinder : Wieland, Heinrich, Dr.
Wiesenweg 2
D-3401 Waake (DE)

(74) Vertreter : von Kreisler, Alek, Dipl.-Chem. et al
Deichmannhaus am Hauptbahnhof
D-5000 Köln 1 (DE)

## Beschreibung

Die Erfindung betrifft ein Verfahren sowie eine Vorrichtung zur selektiven extrakorporalen Abtrennung pathologischer und/oder toxischer Blutbestandteile nach Präzipitation unter Verwendung von Filterkerzen.

Der Oberbegriff des Verfahrens nach Anspruch 3 geht aus Artificial Organs, Band 4, Nr 3, August 1980, Seiten 205 bis 207 : « On-line separation of macromolecules by membrane filtration with cryogelation » hervor.

Der Oberbegriff der Vorrichtung nach Anspruch 10 ist der GB-A-2 020 570 zu entnehmen.

Die Fortschritte der letzten Jahre auf dem Gebiet der Analytik des Lipoproteinsystems des menschlichen Körpers haben ergeben, daß eine hohe Plasmacholesterinkonzentration und damit das Risiko einer frühzeitigen Arteriosklerose, speziell der Koronarsklerose, im wesentlichen auf das Vorhandensein hoher Konzentrationen cholesterinreicher β-Lipoproteine im Körper zurückzuführen ist. Im Blut des Menschen finden sich normalerweise ca. 70 bis 80 % des gesamten Cholesterins an β-Lipoproteine (low density lipoproteins, LDL) gebunden. In Krankheitsprozessen, die mit einem gestörten Fettstoffwechsel bzw. erhöhten Plasmalipidkonzentrationen einhergehen, kann sich der prozentuale Anteil des an LDL gebundenen Cholesterins am Gesamtcholesterin sogar noch erhöhen. Eine Hypercholesterinämie ist daher in der Regel durch eine Hyper-β-Lipoproteinämie hervorgerufen.

Therapeutische Bemühungen, die Konzentration an β-Lipoproteinen effektiv zu senken, waren bisher allesamt unbefriedigend. Eine medikamentöse Beeinflussung ist vor allen Dingen bei den genetischen Formen von Fettstoffwechselstörungen äußerst schwierig.

Die mechanische Abtrennung von β-Lipoproteinen aus dem Blut wurde bisher auf zwei Wegen versucht : Elimination von LDL aus dem Blut mit Hilfe spezifischer Antikörper, die an eine Matrix gekoppelt waren ; kompletter Austausch des gesamten Blutwassers durch sogenannte Plasmapherese.

Die für das erste Verfahren verwendeten Antikörper wurden durch Immunisierung von Schafen oder Kaninchen gewonnen. Dieses Verfahren (W. Stoffel und Th. Demant ; Selective Removal of Apolipoprotein B — Containing Serum Lipoproteins from Blood Plasma, Proc. Nat. Acad. Sci. USA 78, 611 (1981)) hat den Nachteil einer geringen Effizienz, was schon zu einer Änderung des Behandlungsvorgehens nach diesem Verfahren geführt hat und zudem besteht theoretisch die Gefahr, daß die Anwendung von in einem Tier erzeugter Antikörper auf lange Sicht immunologische Komplikationen bei den behandelten Personen auslösen könnte.

Bei der Plasmapherese, die zu einem kompletten Austausch des gesamten Blutwassers führt, wird zwar der Gehalt an β-Lipoproteinen der befallenen Patienten gesenkt, gleichzeitig werden aber im Zuge der bisher üblichen Verfahren aufgrund der apparativen Eigenheiten auch jene Lipoproteine aus dem Blut eliminiert, die der Arteriosklerose entgegenwirken (high density lipoproteins, HDL). Hinzu kommt, daß auch alle anderen Proteine des Plasmas, eingeschlossen Gerinnungsfaktoren, Globuline und Hormone, mit eliminiert werden. Dennoch hat sich dieses Verfahren bisher für ganz bestimmte Fälle von Hyper-β-Lipoproteinämie als brauchbar erwiesen.

Um selektiv und mit hoher Kapazität β-Lipoproteine aus dem Blut bzw. Blutplasma zu entfernen, werden — wie in den DE-OSen 31 35 814 und 32 17 925 beschrieben — auf bekannten Wegen die korpuskulären Bestandteile des Blutes abgetrennt und das verbleibende Plasma mit Heparin in einem geeigneten Puffer, z. B. Acetat-Zitrat-Puffer, versetzt und der gebildete β-Lipoprotein-Heparin-Komplex am isoelektrischen Punkt bei einem pH-Wert von 5,05 bis 5,25 ausgefällt und anschließend abgetrennt.

Zahlreiche andere Krankheiten sind ebenfalls von einem Anstieg des Gehalts an Antikörpern oder makromolekularen Spezies pathogener Natur begleitet, der ebenfalls durch Plasmapherese erfolgreich gesenkt werden konnte. Dazu werden die toxischen Spezies durch Präzipitation selektiv ausgefällt. Beispiele solcher Spezies sind Globuline, wie z. B. der Rheumafaktor, Bence-Jones-Körper oder auch andere pathogene Makromoleküle.

Zur Abtrennung derartiger hochmolekularer Spezies aus dem Blutplasma werden verschiedene Methoden beschrieben : So offenbart die CH-A-6 26 256 eine Vorrichtung zur kontinuierlichen, extrakorporalen Abtrennung derartiger Substanzen aus dem Blut, die in einer Filtriervorrichtung mit einem die makromolekularen Spezies adsorbierenden Mittel besteht. Der Adsorber enthält unlöslich gemachte Enzyme, Antigene oder Antikörper, ist jedoch zu einer selektiven Abtrennung bestimmter Spezies neben verwandten, im Plasma ebenfalls vorhandenen besonders unter unphysiologischen Bedingungen, wie sie bei einer Cryopräzipitation oder nach Hitzedenaturierung auftreten, nicht in der Lage.

Filtrationsmethoden unter Verwendung von Membran- oder Kapillarfiltern werden z. B. in der EP-A-0 041 350 beschrieben. In einer sogenannten Filtrationskaskade wird aus dem Blut in einer ersten Filterstufe das Plasma, das die Makromoleküle enthält abgetrennt und dann daraus bei physiologischen Temperaturen oder unter Kühlung in einer zweiten Filtrationsstufe die Makromoleküle entfernt. Der Porendurchmesser der zweiten Membran wird mit nominell 0,01 bis 0,2 μm angegeben.

Nachteil derartiger Membran- und Kapillarfilter ist, daß sie zur Abtrennung größerer Mengen von Präzipitaten zu geringe Flächen besitzen und die Porendurchmesser aus Membranstabilitätsgründen zu gering sind. Ferner verstopfen derartige Filter durch die Präzipitate und durch mitgerissenes Fibrin sehr schnell, besonders dann, wenn der Filtrationsvorgang unter Druck abläuft. Häufig wird damit ein Filterwechsel im extrakorporalen Kreislauf mit allen damit verbundenen medizinischen und technischen Komplikationen erforderlich.

Die vorliegende Erfindung stellt sich daher die Aufgabe, ein Verfahren und eine Vorrichtung zu entwickeln, die es gestatten, selektiv und mit hoher Kapazität pathologische und/oder toxische höhermolekulare Spezies aus dem Blut, d. h. dem Vollserum oder Bestandteilen des Blutes wie Plasma, im extrakorporalen Kreislauf zu entfernen. Dabei sollten Filter Verwendung finden, die, nach Wahl einer geeigneten und in weiten Grenzen variierbaren Porenweite, aus einer großen Menge Plasma größere Präzipitatmengen vollständig zurückhalten, dem Plasma nur geringen Widerstand entgegensetzen und im Verlauf des Filtrationsvorganges nicht verstopfen.

Gelöst wird die Aufgabe durch die Verwendung von Filterkerzen in einem für die Abtrennung der genannten Spezies geeigneten Verfahren und einer Vorrichtung.

Die Erfindung betrifft ein Verfahren zur selektiven, extrakorporalen Abtrennung von durch Zugabe von Fällungsmitteln und/oder chemischen Reagenzien oder Denaturierung durch Abkühlen oder Erhitzen erzeugten Präzipitaten makromolekularer pathologischer und/oder toxischer Spezies aus Blut oder Bestandteilen des Blutes wie Vollserum oder Plasma, wobei eine Plasmafraktion über ein Kapillar- oder Membranplasmafilter oder dem letzten Filter einer Kaskadenschaltung mehrerer derartiger Filter mit molekularen Ausschlußgrenzen von 50 000 bis 3 000 000 Dalton kontinuierlich aus Patientenblut oder Fraktionen davon erzeugt wird und durch Zugabe von Fällungsmitteln und/oder chemischen Reagenzien oder Denaturierung durch Abkühlen oder Erhitzen erzeugte Präzipitate makromolekularer pathologischer und/oder toxischer Spezies enthält, wobei das Verfahren nicht als therapeutische Behandlung oder Diagnostizierverfahren am menschlichen oder tierischen Körper durchgeführt wird, das dadurch gekennzeichnet ist, daß man diese Plasmafraktion

a) drucklos durch ein zylinderförmiges, sterilisiertes, mit einer Filterkerze bestücktes Gehäuse führt, wobei das zugeführte Plasma die Filterfläche von außen umströmt, das durch Filtrieren gereinigte Plasma ins Innere der Kerze dringt und das Präzipitat im Filtergewebe zurückgehalten wird,

b) das gereinigte Plasma über einen zentralen Ablaufstutzen des zylindrischen Gefäßes abführt und

c) das gereinigte Plasma, gegebenenfalls nach Abtrennung überschüssiger Reagenzien bzw. Korrektur der Temperaturänderungen, mit dem Blut- oder Plasmastrom aus dem vorgeschalteten Filter vereinigt.

Die Erfindung betrifft außerdem eine Vorrichtung zur selektiven extrakorporalen Abtrennung von durch Zugabe von Fällungsmitteln und/oder chemischen Reagenzien oder Denaturierung durch Abkühlen oder Erhitzen erzeugten Präzipitaten makromolekularer pathologischer und/oder toxischer Spezies aus Blut oder Bestandteilen des Blutes wie Vollserum oder Plasma, wobei die Vorrichtung ein sterilisierbares zylinderförmiges Gehäuse (1) aus Kunststoff oder Glas mit An- und Ablaufstutzen umfaßt, das dadurch gekennzeichnet ist, daß das Gehäuse mit einem Gewinde am oberen und unteren Rohrende und damit kompatiblen Deckeln mit einem tangential angebrachten Zu- und einem zentral angebrachten Ablaufstutzen ausgestattet ist und eine Filterkerze enthält, die eine effektive Filterfläche von 0,2 bis 2 m² und einen mittleren Porendurchmesser von 0,2 bis 2 μm hat und die gleiche Länge wie das sie umgebende zylindrische Gehäuse aufweist.

Die Erfindung betrifft schließlich die Verwendung von Filterkerzen von 0,2 bis 2 m² effektiver Filterfläche, einer Länge von 10 bis 50 cm sowie einem mittleren Porendurchmesser von 0,2 bis 2 μm in sterilisierbaren zylinderförmigen Gehäusen aus Kunststoff oder Glas für die selektive extrakorporale Abtrennung von Präzipitaten makromolekularer pathologischer und/oder toxischer Spezies aus Blut oder Bestandteilen des Blutes wie Vollserum oder Plasma.

Die für die erfindungsgemäße Vorrichtung verwendeten Filterkerzen finden üblicherweise Verwendung in technischen Filtrationsprozessen, besonders in der Reinigung von Trinkwasser und der Herstellung von ultrareinen Wässern für besondere Verwendungszwecke. Sie weisen einen mittleren Porendurchmesser von 0,2 bis 2 μm, vorzugsweise 0,4 bis 1 μm auf, was sie einerseits befähigt, die gefällten pathologischen und/oder toxischen Spezies als Präzipitat vollständig zurückzuhalten, andererseits jedoch dem Plasma so gut wie keinen Widerstand entgegenzusetzen.

Die effektive Filteroberfläche der verwendeten Filterkerzen liegt bei 0,2 bis 2 m², vorzugsweise 0,8 bis 1,7 m², je nach Länge der verwendeten Filterkerzen, die sich zwischen 10 und 50 cm bewegt. Derart große Oberflächen erweisen sich als geeignet, die gesamte Präzipitatmenge aus 2 l Plasma, die eine typische Menge eines Reinigungsdurchlaufs an einem Patienten ist, aufzunehmen, ohne zu verstopfen.

In der Vorrichtung gemäß der vorliegenden Erfindung wurden beispielsweise NUCLEPORE : eingetragenes Warenzeichen QR Membran-Filterkerzen verwendet, deren Filtrationsmedium aus einer Polycarbonatmembran besteht. Erfindungsgemäß können alle Filterkerzen verwendet werden, die den hier genannten Anforderungen genügen.

Andere Filtrationssysteme, wie z. B. Durchflußzellen der Firma Millipore oder Radialflußzellen der Firma Berghoff, erwiesen sich in ihrer Filtrationskapazität für Präzipitate als viel zu klein und verstopften leicht, was zu einem verlangsamten Plasmafluß, einem Druckanstieg mit begleitender Porenverengung und daraus resultierender verringerter Durchlässigkeit für das Filtrat führte. Dies verschlechtert den Trenneffekt.

Die Vorrichtung gemäß der vorliegenden Erfindung sowie zwei spezielle Ausführungsformen dieser Vorrichtung werden in den Figuren 1 bis 3 gezeigt.

Figur 1 zeigt die erfindungsgemäße Vorrichtung, wie sie z. B. für die Abtrennung von bei Raumtemperatur mit Heparin gefällten Präzipitaten von low-density-Lipoproteinen aus Blut oder Blutplasma verwendet wird.

Figur 2 zeigt eine für die Fällung und nachfolgende Abtrennung pathologischer oder toxischer Blutbestandteile bei niedriger oder erhöhter Temperatur geeignete bevorzugte Ausführungsform der erfindungsgemäßen Vorrichtung.

Figur 3 zeigt eine weitere bevorzugte Ausführungsform der Erfindung, wie sie bei der Abtrennung von nach Hitzedenaturierung gefällten Präzipitaten Verwendung findet.

Die erfindungsgemäße Vorrichtung zur selektiven extrakorporalen Abtrennung der Präzipitate aus Blut oder Blutplasma, die als Filterteil in ein Kaskadenfiltrations-System eingebaut werden kann, besteht aus einem zylinderförmigen Gehäuse 1 aus Kunststoff oder Glas, in das am oberen und unteren Rohrende ein Gewinde eingeschnitten ist. Der Kunststoff- bzw. Glaszylinder ist am oberen und unteren Rohrende mit je einem aufschraubbaren Deckel 2 verschlossen, der mit einer Ultraschallschweißung luftdicht und steril das jeweilige Rohrende verschließt. Die Deckel sind so ausgebildet, daß sie je einen tangential angebrachten und je einen zentral angebrachten Zu- bzw. Ablaufstutzen 5, 6, bzw. 7, 8 enthalten, über die der Zu- bzw. Ablauf des Plasmas bzw. Präzipitats erfolgt.

Das zylinderförmige Kunststoff- bzw. Glasgehäuse enthält eine in den Maßen genau dem inneren Volumen des Zylinders entsprechende Filterkerze z. B. der Firma NUCLEPORE®, die dergestalt dicht in das Filtergehäuse eingeklebt ist, daß der innere Kern 3 zwischen den zentral angebrachten Stutzen der Deckel 6 bzw. 8 liegt, während der Filterkörper 4 in Höhe der tangential angebrachten Stutzen 5 bzw. 7 liegt.

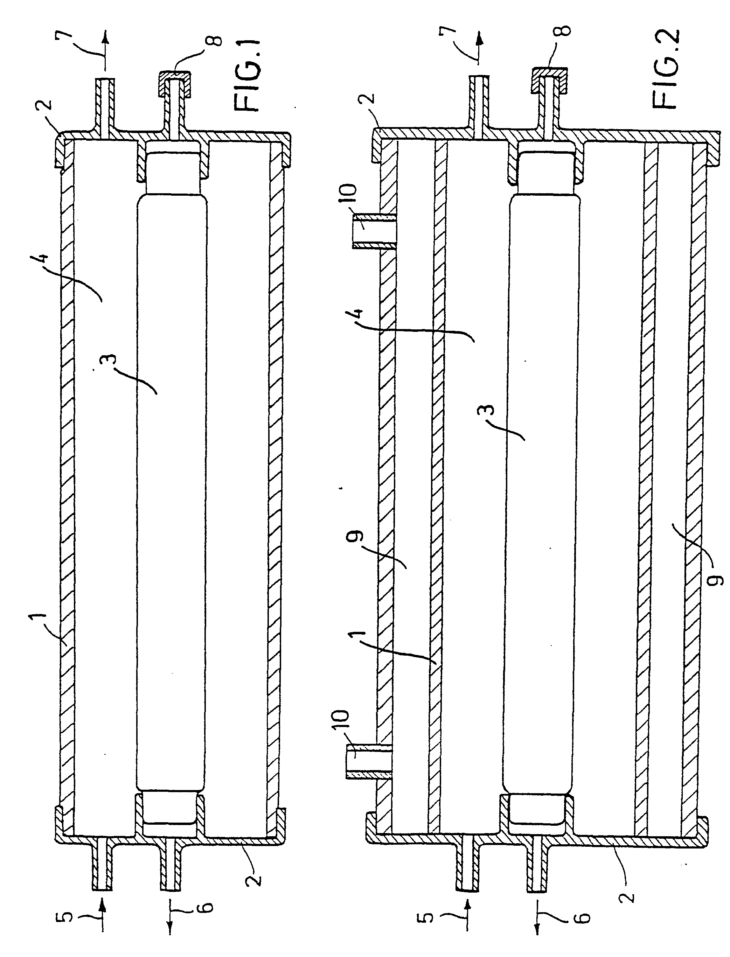

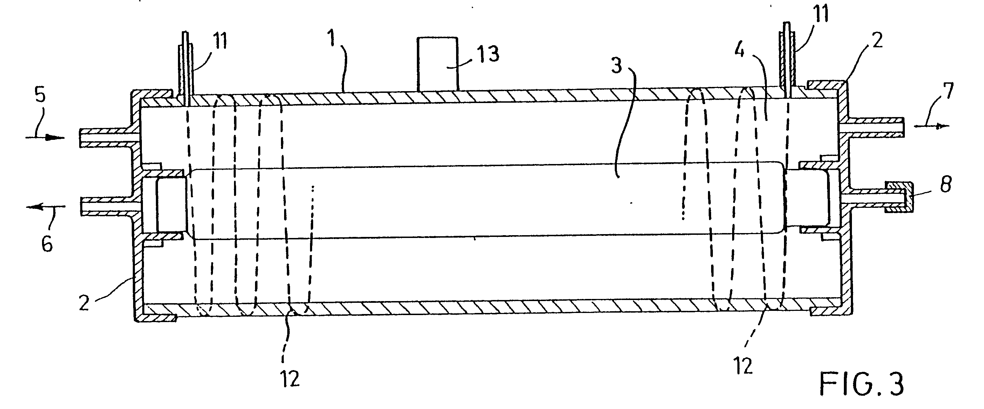

In speziellen Ausführungsformen der erfindungsgemäßen Vorrichtung (Fig. 2) für besondere Zwecke, z. B. die Cryopräzipitation oder Hitzedenaturierung, ist das zylinderförmige Kunststoffbzw. Glasgehäuse von einem Thermostatisier-Mantel 9 umgeben, durch den über die Zu- bzw. Ablaufstutzen 10 eine beliebige Kühl- oder Heizflüssigkeit geleitet werden kann. In einer weiteren Ausführungsform der Vorrichtung (Fig. 3) kann der Zylinder mit Hilfe von eingebauten Heizdrähten 12 erhitzt werden, um eine Denaturierung von im Plasma enthaltenen Bestandteilen durch Hitze zu erreichen. Die Heizdrähte 12 werden über, zwei Steckkontakte 11 mit Strom versorgt, die so angebracht sind, daß sie, wie auch die Befestigungskupplung 13, ein direktes Einstecken des Filtergehäuses in die Gehäusewand z. B. eines Monitors erlauben.

Die verwendeten NUCLEPORE : eingetragenes Warenzeichen QR-Filterkerzen, die beschriebene Vorrichtung gemäß der Erfindung wie auch die Verbindungen zu dem übrigen Kreislaufsystem zur extrakorporalen Abtrennung von Präzipitaten aus Blut oder Blutplasma sind so gestaltet, daß sie ein Arbeiten unter sterilen Bedingungen, das in solchen Fällen unabdingbar ist, ermöglichen. Dazu gehört, daß alle Materialien so gewählt sind, daß sie eine Sterilisation unter üblichen Bedingungen ermöglichen.

Ein wesentlicher Vorteil des erfindungsgemäßen Verfahrens gegenüber den bekannten Verfahren nach dem Stand der Technik ist es, daß im Vergleich zu Kapillarfiltern kein seitlicher Ablaufstutzen am Zylindermantel zum Abziehen des gereinigten Plasmas mittels Unterdruck notwendig ist. Aufgrund der konstruktiven Gegebenheiten der Filterkerzen umströmt das durch den Stutzen 5 zugeführte Plasma die Filterfläche der Filterkerze 4 von außen. Das Filtrat dringt ohne Anlegen eines äußeren Unterdrucks allein durch Schwerkraft ins Innere der Kerze 3 und wird über einen zentralen Ablaufstutzen 6 weggeführt. Das Präzipitat verbleibt im Filtergewebe. Ein Teil des einfließenden Plasmas, das nicht durch Schwerkraft abfiltriert wird, kann über einen weiteren peripheren, am Deckel der Auslaufseite des Filters angebrachten Stutzen 7 abgeführt und gegebenenfalls rezirkulierend mit dem Plasmaeinlauf vereinigt werden. Der Stutzen 8 bleibt dabei verschlossen.

Soll z. B. zur Abtrennung des Rheumafaktors das Plasma im Zuge einer Cryopräzipitation auf niedrige Temperaturen gekühlt werden, um ein Ausfallen der abzutrennenden Spezies zu erreichen, so leitet man eine geeignete Temperierflüssigkeit, vorzugsweise Wasser, über einen Stutzen 10 in den Thermostatisiermantel 9 eines Doppelmantelgefäßes gemäß Fig. 2, was eine Abkühlung des Plasmas auf eine durch einen Thermostaten vorgegebene Temperatur zur Folge hat. Das gereinigte Plasma, das über den Abzugstutzen 6 aus dem Gehäuse geführt wird, muß dann vor der Rückführung in die Blutbahn des Patienten wieder auf Körpertemperatur erwärmt werden.

Zur Abtrennung von durch Hitzedenaturierung gefällten Bestandteilen des Blutplasmas, z. B. Bence-Jones-Körpern, wird das Plasma gemäß einer weiteren Ausführungsform des Verfahrens in einen mit einer Filterkerze versehenen Kunststoff- oder Glaszylinder entsprechend der obigen Beschreibung geleitet, dessen Mantel mit Hilfe eines Heizdrahtes auf eine vorgegebene Temperatur erwärmt werden kann. Die Heizdrähte 12 des Heizmantels werden über Steckkontakte 11 mit Strom versorgt; über ein Regelsystem läßt sich die erreichte Temperatur ebenfalls genau kontrollieren. Das durch den Ablaufstutzen 6 geführte gereinigte Plasma muß entsprechend vor der Rückführung in den Patientenkreislauf auf Körpertemperatur gekühlt werden.

Die weiteren Vorteile des Verfahrens gemäß der Erfindung liegen also darin, daß je nach den speziellen Anforderungen des jeweiligen Abtrenn-Vorgangs ganz bestimmte, kontrollierte Bedingungen eingestellt und eingehalten werden können, ohne daß dabei die sonstigen Vorteile des Verfahrens, nämlich eine fast drucklose Filtration über Filtersysteme mit sehr großer Oberfläche ohne die Gefahr des Verstopfens der Filter und allen damit verbundenen Komplikationen, aufgegeben werden.

Die Erfindung wird durch die nachstehenden Beispiele näher erläutert.

Beispiel 1

Das Blut eines Patienten mit familiärer Hypercholesterinämie (homozygot) mit dem Ausgangscholesterinwert von 416 mg/dl bzw. LDL-Cholesterinwert von 368 mg/dl war von β-Lipoproteinen zu befreien.

Verwendet wurde ein einfaches Zylindergehäuse gemäß Fig. 1, das eine NUCLEPORE : eingetragenes Warenzeichen QR-Filterpatrone mit einem mittleren Porendurchmesser von 0,4 μm enthielt.

2 l Blutplasma des Patienten wurden im Volumenverhältnis 1/1 mit 2 l 0,2 M Natriumacetatpuffer (pH : 4,86) verdünnt, der 50 000 E/l Heparin enthielt, woraus ein pH-Wert des Gemisches von 5,12 resultierte.

Die Heparin-Puffer-Plasmamischung wurde dem Verfahren gemäß der vorliegenden Erfindung unterworfen, wobei das Plasma innerhalb von 30 Minuten vollständig von low-density-Lipoproteinen befreit wurde.

Bei dieser nur in seltenen Fällen in dieser Höhe auftretenden LDL-Menge wurde noch kein Druckanstieg am Filter beobachtet. Da Filtrat blieb bis zum Schluß klar. Das klare Filtrat enthielt zu keinem Zeitpunkt low-density-Lipoproteine.

Beispiel 2

Entsprechend Beispiel 1 wurde Plasma aus Patientenblut, das den Rheumafaktor und « $C_{1q}$ binding immune complexes » enthielt, der Cryopräzipitation unterworfen. Dazu wurde das Plasma in die erfindungsgemäße Vorrichtung gemäß Fig. 2 eingeleitet, deren Thermostatisiermantel auf 4 °C gekühlt worden war. Als Filterkerze wurde eine NUCLEPORE : eingetragenes Warenzeichen QR-Filterkerze verwendet, deren Porenweite 0,4 μm betrug. Nach der Filtration enthielt das gereinigte Plasma unter 5 % der ursprünglich vorhandenen Rheumafaktoren und « $C_{1q}$ binding immune complexes ».

Beispiel 3

Die Abtrennung von Bence-Jones-Körpern aus Blutplasma erfolgte nach Denaturieren durch Erhitzen auf 50 °C in einer Vorrichtung gemäß Fig. 2, deren Thermostatisiermantel auf 50 °C erhitzt worden war. Zur Verwendung kamen NUCLEPORE : eingetragenes Warenzeichen QR-Filterkerzen mit einem mittleren Porendurchmesser von 0,4 μm. Das Plasma war nach der Filtration frei von Denaturierungsprodukten. Ein Verstopfen der Filter wurde nicht beobachtet.

Beispiel 4

Die Abtrennung von Bence-Jones-Körpern aus Blutplasma erfolgte nach Denaturieren durch Erhitzen auf 50 °C in einer Vorrichtung gemäß Fig. 3. Zur Verwendung kamen NUCLEPORE : eingetragenes Warenzeichen QR-Filterkerzen mit einem mittleren Porendurchmesser von 0,4 μm.

Das Plasma war nach der Filtration frei von Denaturierungsprodukten. Ein Verstopfen der Filter wurde nicht beobachtet.

**Patentansprüche**

1. Verwendung von Filterkerzen mit 0,2 bis 2 m² effektiver Filterfläche, einer Länge von 10 bis 50 cm sowie einem mittleren Porendurchmesser von 0,2 bis 2 μm, in sterilisierbaren, zylinderförmigen Gehäusen aus Kunststoff oder Glas für die selektive extrakorporale Abtrennung von Präzipitaten makromolekularer pathologischer und/oder toxischer Spezies aus Blut oder Bestandteilen des Blutes, wie Vollserum oder Plasma.

2. Verwendung von Filterkerzen nach Anspruch 1 mit 0,8-1,7 m² effektiver Filterfläche, einer Länge von 10-50 cm sowie einem mittleren Porendurchmesser von 0,4-1 μm.

3. Verfahren zur selektiven extrakorporalen Abtrennung von durch Zugabe von Fällungsmitteln und/oder chemischen Reagenzien oder Denaturierung durch Abkühlung oder Erhitzen erzeugten Präzipitaten makromolekularer pathologischer und/oder toxischer Spezies aus Blut oder Bestandteilen des Blutes wie Vollserum oder Plasma, wobei eine Plasmafraktion über ein Kapillar- oder Membranplasmafilter oder den letzten Filter einer Kaskadenschaltung mehrerer derartiger Filter mit molekularen Ausschlußgrenzen von 50 000 bis 3 000 000 Dalton kontinuierlich aus Patientenblut oder Fraktionen davon erzeugt wird und durch Zugabe von Fällungsmitteln und/oder chemischen Reagenzien oder Denaturierung durch Abkühlen oder Erhitzen erzeugte Präzipitate makromolekularer pathologischer und/oder toxischer Spezies enthält, wobei das Verfahren nicht als therapeutische Behandlung oder Diagnostizierverfahren am menschlichen oder tierischen Körper durchgeführt wird, dadurch gekennzeichnet, daß man diese Plasmafraktion

a) drucklos durch ein zylinderförmiges, sterilisiertes, mit einer Filterkerze bestücktes Gehäuse führt, wobei das zugeführte Plasma die Filterfläche von außen umströmt, das durch Filtration gereinigte Plasma ins Innere der Kerze dringt und das Präzipitat im Filtergewebe zurückgehalten wird,

b) das gereinigte Plasma über einen zentralen Ablaufstutzen des zylindrischen Gefäßes abführt und

c) das gereinigte Plasma, gegebenenfalls nach Abtrennung überschüssiger Reagenzien bzw. Korrektur der Temperaturänderungen, mit dem Blut- oder Plasmastrom aus dem vorgeschalteten Filter vereinigt.

4. Verfahren nach Anspruch 3, dadurch gekennzeichnet, daß man die Filtration bei Körpertemperatur in einem zylinderförmigen Gehäuse aus Kunststoff oder Glas durchführt.

5. Verfahren nach Anspruch 3, dadurch gekennzeichnet, daß man die Filtration bei Temperaturen unterhalb der Körpertemperatur bis zu + 4ºC in einem mit einem Thermostatisiermantel umgebenen zylinderförmigen Gehäuse aus Kunststoff oder Glas durchführt, der mit einer Temperierflüssigkeit, bevorzugt Wasser, gekühlt wird.

6. Verfahren nach Anspruch 3, dadurch gekennzeichnet, daß man die Filtration bei Temperaturen oberhalb der Körpertemperatur bis zu 60 ºC in einem mit einem Thermostatisiermantel umgebenen zylinderförmigen Gehäuse aus Kunststoff oder Glas durchführt, der mit einer Temperierflüssigkeit, bevorzugt Wasser, geheizt wird.

7. Verfahren nach Anspruch 3, dadurch gekennzeichnet, daß man die Filtration bei Temperaturen oberhalb Körpertemperatur bis 60 ºC in einem zylindrischen Gehäuse aus Glas oder Kunststoff durchführt, welches in der Gehäusewand Heizdrähte und Kontaktstifte enthält, die eine Regelung der Temperatur über einen Monitor ermöglichen.

8. Verfahren nach Ansprüchen 3 bis 7, dadurch gekennzeichnet, daß als Filtermedium Filterkerzen mit einer effektiven Filterfläche von 0,2 bis 2 m², einer Länge von 10 bis 50 cm und einem mittleren Porendurchmesser von 0,2 bis 2 μm verwendet werden.

9. Verfahren nach Ansprüchen 3 bis 8, dadurch gekennzeichnet, daß der Teil des Plasmas, der nicht durch Schwerkraft abfiltriert wird, über einen peripheren, am Deckel der Auslaufseite des Filtergehäuses angebrachten Stutzen weggeführt wird und rezirkulierend mit dem Plasmaeinlauf vereinigt wird.

10. Vorrichtung zur selektiven extrakorporalen Abtrennung von durch Zugabe von Fällungsmitteln und/oder chemischen Reagenzien oder Denaturierung durch Abkühlen oder Erhitzen erzeugten Präzipitaten makromolekularer pathologischer und/oder toxischer Spezies aus Blut oder Bestandteilen des Blutes, wie Vollserum oder Plasma, wobei die Vorrichtung ein sterilisierbares, zylinderförmiges Gehäuse (1) aus Kunststoff oder Glas mit Zulaufstutzen und Ablaufstutzen umfasst, dadurch gekennzeichnet, daß das Gehäuse mit einem Gewinde am oberen und unteren Rohrende und damit kompatiblen Deckeln (2) mit je einem tangential angebrachten Zu- und einem zentral angebrachten Ablaufstutzen ausgestattet ist und eine Filterkerze enthält, die eine effektive Filterfläche von 0,2 bis 2 m² und einen mittleren Porendurchmesser von 0,2 bis 2 μm hat und die gleiche Länge wie das sie umgebende zylindrische Gehäuse aufweist.

11. Vorrichtung nach Anspruch 10, dadurch gekennzeichnet, daß sie gegebenenfalls von einem Thermostatisiermantel (9) mit Zu- und Abläufen (10) für die Versorgung mit Temperierflüssigkeit umgeben ist.

12. Vorrichtung nach Anspruch 10, dadurch gekennzeichnet, daß der äußere Mantel gegebenenfalls Heizdrähte (12) mit Steckkontakten (11) zur Stromversorgung aufweist.

## Claims

1. Use of filter candles having from 0.2 to 2 m² of effective filter surface area, a length of from 10 to 50 cm and an average pore diameter of from 0.2 to 2 μm, in sterilizable cylindrical housings made of plastics or glass, for the selective extracorporeal separation of precipitates of macromolecular pathologic and/or toxic species from blood or blood constituents such as full serum or plasma.

2. The use of filter candles according to claim 1 which have from 0.8 to 1.7 m² of effective filter surface area, a length of from 10 to 50 cm and an average pore diameter of from 0.4 to 1 μm.

3. A process for the selective extracorporeal separation of precipitates of macromolecular pathologic and/or toxic species from blood or blood constituents such as full serum or plasma, which precipitates have been produced by the addition of precipitating agents and/or chemical reagents and or denaturation by cooling or heating, wherein a plasma fraction is continuously produced from patients' blood or fractions thereof through a capillary or membrane plasma filter or the last filter of a cascade arrangement of several such filters having molecular exclusion limits of from 50 000 to 3 000 000 Dalton and contains precipitates of macromolecular pathologic and/or toxic species produced by the addition of precipitating agents and/or chemical reagents and or denaturation by cooling or heating, said process being not carried out as a therapeutic treatment or diagnostic procedure on the human or animal body, characterized in that

a) said plasma fraction is pressureless passed through a cylindrical sterilizable housing provided with a filter candle, whereby the supplied plasma flows around the filter area from outside, the plasma purified by the filtration penetrates into the interior of the candle, and the precipitate is retained in the filter tissue,

b) the purified plasma is removed by a central discharge port of the cylindrical vessel, and

c) the purified plasma, if required or desired after the separation of excessive reagents or corrections of the temperature changes is combined with the blood or plasma stream from the preceding filter.

4. The process according to claim 3, characterized in that the filtration is carried out at body temperature in a cylindrical housing made of plastics or glass.

5. The process according to claim 3, characterized in that the filtration is carried out at temperatures below the body temperature to + 4 ºC in a cylindrical housing made of plastics or glass surrounded by a thermostated jacket which is cooled with a thermostating liquid, and preferably water.

6. The process according to claim 3, characterized in that the filtration is carried out at temperatures above the body temperature to 60 °C in a cylindrical housing made of plastics or glass surrounded by a thermostated jacket which is heated with a thermostating liquid, and preferably water.

7. The process according to claim 3, characterized in that the filtration is carried out at temperatures above the body temperature to 60 °C in a cylindrical housing made of plastics or glass which, in the housing wall, contains heating wires and contact pins which allow the temperature to be controlled via a monitor.

8. The process according to claims 3 to 7, characterized in that filter candles having from 0.2 to 2 m² of effective filter surface area, a length of from 10 to 50 cm and an average pore diameter of from 0.2 to 2 μm are used as the filter medium.

9. The process according to claims 3 to 8, characterized in that that part of the plasma which is not filtered off under the action of gravity is removed through a peripheral port connected to the lid of the discharge side of the filter housing and is recycled to and combined with the plasma feed.

10. A device for the selective extracorporeal separation of precipitates of macromolecular pathologic and/or toxic species from blood or blood constituents such as full serum or plasma, said precipitates having been produced by the addition of precipitating agents and/or chemical reagents and or denaturation by cooling or heating, said device comprising a sterilizable cylindrical housing (1) made of plastics or glass having inlet and outlet ports, characterized in that the housing has been provided with threads at the upper and lower pipe ends and with lids (2) compatible therewith each comprising a tangentially disposed inlet port and a centrally disposed outlet port and contains a filter candle having from 0.2 to 2 m² of effective filter surface area and an average pore diameter of from 0.2 to 2 μm and the same length as the cylindrical housing surrounding it.

11. The device according to claim 10, characterized in that it is optionally surrounded by a thermostated jacket (9) having inlet and outlet ports (10) for the supply with thermostated liquid.

12. The device according to claim 10, characterized in that the outer jacket optionally comprises heating wires (12) with plug contacts (11) for electric power supply.

## Revendications

1. Utilisation de bougies filtrantes ayant 0,2 à 2 m² de surface efficace de filtre, une longueur de 10 à 50 cm ainsi qu'un diamètre moyen des pores de 0,2 à 2 μm, dans des boîtiers cylindriques stérilisables réalisés en une matière plastique ou en verre, pour la séparation extracorporelle sélective de précipités d'espèces macromoléculaires pathologiques et/ou toxiques à partir du sang ou de constituants du sang, comme du sérum entier ou du plasma.

2. Utilisation de bougies filtrantes selon la revendication 1, ayant une surface efficace de filtre de 0,8 à 1,7 m², une longueur de 10 à 50 cm ainsi qu'un diamètre moyen des pores de 0,4 à 1 μm.

3. Procédé de séparation extra-corporelle sélective de précipités produits par addition d'agents de précipitation et/ou de réactifs chimiques ou par dénaturation par refroidissement ou chauffage d'espèces macromoléculaires pathologiques et/ou toxiques à partir du sang ou de constituants du sang, comme du sérum entier ou du plasma, procédé selon lequel on obtient en continu, à partir du sang d'un patient ou de fractions de ce sang, une fraction de plasma par l'intermédiaire d'un filtre à capillaire ou à membrane pour plasma ou par l'intermédiaire du dernier filtre d'un agencement en cascade comportant plusieurs filtres de ce genre, ayant des limites moléculaires d'exclusion allant de 50 000 à 3 000 000 de Dalton et qui contient des précipités d'espèces macromoléculaires, pathologiques et/ou toxiques, produits par addition d'agents de précipitation et/ou de réactifs chimiques ou par dénaturation par refroidissement ou par chauffage, le procédé n'étant pas conduit à titre de traitement thérapeutique ou de procédé pour diagnostic sur des corps humains ou animaux, procédé caractérisé en ce qu'on achemine

a) cette fraction de plasma, sans application d'une pression, dans un boîtier de forme cylindrique, stérilisé, équipé d'une bougie filtrante, le plasma introduit passant de l'extérieur à travers la surface filtrante, en ce que le plasma purifié par la filtration pénètre à l'intérieur de la bougie et en ce que le précipité est retenu sur l'étoffe filtrante,

b) le plasma purifié est évacué du boîtier cylindrique par un raccord central de sortie, et

c) le plasma purifié, éventuellement après séparation des réactifs excédentaires ou correction des variations de la température, est combiné avec le courant de sang ou de plasma provenant du filtre placé en amont.

4. Procédé selon la revendication 3, caractérisé en ce qu'on conduit la filtration à la température du corps, dans un boîtier de forme cylindrique, réalisé en une matière plastique ou en verre.

5. Procédé selon la revendication 3, caractérisé en ce qu'on conduit la filtration à des températures inférieures à la température du corps, allant jusqu'à + 4 °C, dans un boîtier de forme cylindrique, en matière plastique ou en verre, entouré d'une enveloppe thermostatée et qui est refroidi à l'aide d'un liquide de mise en température, de préférence à l'aide d'eau.

6. Procédé selon la revendication 3, caractérisé en ce qu'on conduit la filtration à des températures supérieures à la température du corps et allant jusqu'à 60 °C dans un boîtier cylindrique, en matière plastique ou en verre, entouré d'une enveloppe thermostatée, qui est chauffée à l'aide d'un liquide de mise en température, de préférence de l'eau.

7. Procédé selon la revendication 3, caractérisé en ce qu'on conduit la filtration à des températures supérieures à la température du corps et pouvant aller jusqu'à 60 °C dans un boîtier cylindrique en verre ou en matière plastique, qui contient dans la paroi de ce boîtier des fils de chauffage et des fiches de contact permettant une régulation de température à l'aide d'un appareil de surveillance ou moniteur.

8. Procédé selon les revendications 3 à 7, caractérisé en ce qu'on utilise comme milieu filtrant des bougies filtrantes ayant une surface efficace de filtre de 0,2 à 2 m², une longueur de 10 à 50 cm et un diamètre moyen des pores de 0,2 à 2 $\mu$m.

9. Procédé selon les revendications 3 et 8, caractérisé en ce que la partie de plasma, qui n'est pas séparée par filtration sous l'effet de la pesanteur, est enlevée à l'aide d'un raccord périphérique fixé sur le couvercle du côté sortie du boîtier filtrant, et en ce que cette partie est combinée, en recyclage, avec le plasma d'entrée.

10. Dispositif pour une séparation extracorporelle sélective de précipités produits par l'addition d'agents de précipitation et/ou de réactifs chimiques ou par dénaturation par refroidissement ou chauffage d'espèces macro-moléculaires, pathologiques et/ou toxiques à partir du sang ou de constituants du sang, comme du sérum entier ou du plasma, le dispositif comportant un boîtier (1) cylindrique stérilisable, dans une matière plastique ou dans du verre, avec des raccords d'entrée et des raccords de sortie, dispositif caractérisé en ce que le boîtier comporte un filetage à l'extrémité supérieure et à l'extrémité inférieure du tube et des couvercles (2) compatibles avec ces filetages et comportant chacun un raccord tangentiel d'admission et un raccord central de sortie et en ce que le boîtier contient une bougie filtrante présentant une surface effective de filtre de 0,2 à 2 m² et un diamètre moyen des pores de 0,2 à 2 $\mu$m et présentant la même longueur que le boîtier cylindrique entourant la bougie.

11. Dispositif selon la revendication 10, caractérisé en ce qu'il est éventuellement entouré d'une enveloppe (9) thermostatée comportant des entrées et sorties (10) pour l'alimentation en un liquide de réglage de température.

12. Dispositif selon la revendication 10, caractérisé en ce que l'enveloppe externe présente éventuellement des fils (12) de chauffage comportant des fiches (11) de contact pour l'alimentation en courant électrique.

FIG.1

FIG.2

FIG.3